# EUROPEAN PATENT APPLICATION

(11) **EP 4 155 414 A1**
(43) Date of publication of application: **29.03.2023**
(21) Application number: 21921679.3
(22) Date of filing: 20.12.2021
(51) Int. Cl.: C12Q 1/68, C12N 15/00

(54) **INDEL MOLECULAR MARKER OF ULTRASONIC MUTAGENESIS SALMONELLA TYPHIMURIUM HISD GENE AND USE THEREOF**

(30) Priority: 09.08.2021 CN 202110909368
(71) Applicant: Jiangsu University, Zhenjiang, Jiangsu 212013 (CN)
(72) Inventor: MA, Haile, Zhenjiang Jiangsu 212013 (CN); RUAN, Siyu, Zhenjiang Jiangsu 212013 (CN); LI, Yunliang, Zhenjiang Jiangsu 212013 (CN); JI, Yaoyong, Zhenjiang Jiangsu 212013 (CN); ZHOU, Anqi, Zhenjiang Jiangsu 212013 (CN); XIE, Pengfei, Zhenjiang Jiangsu 212013 (CN); ZHAO, Bobo, Zhenjiang Jiangsu 212013 (CN); LIU, Xiaoshuang, Zhenjiang Jiangsu 212013 (CN); XU, Yaxuan, Zhenjiang Jiangsu 212013 (CN); WU, Yanling, Zhenjiang Jiangsu 212013 (CN)
(74) Representative: Herrmann, Uwe
(86) International application number: PCT/CN2021/139459
(87) International publication number: WO 2023/015810

(57) **Abstract**

The present disclosure provides an insertion and deletion (InDel) molecular marker of a *hisD* gene of ultrasonically-mutagenized *Salmonella typhimurium* (*S. typhimurium*) and use thereof, and belongs to the field of molecular biology. In the present disclosure, through ultrasonic mutagenesis to *S. typhimurium* and sequencing, it is found that all InDel mutations occur in a core mutation region of the *hisD* gene. Therefore, the core mutation region is used as the InDel molecular marker to determine an insertion or a deletion of a gene reverse mutation, and the InDel molecular marker can also be used to analyze a relationship between a sequence of the *hisD* gene and a function of a protein encoded thereby.

## Description

### TECHNICAL FIELD

The present disclosure belongs to the technical fields of molecular biology and ultrasonic mutagenesis, and in particular relates to an insertion and deletion (InDel) molecular marker of a *hisD* gene of ultrasonically-mutagenized *Salmonella typhimurium* (*S. typhimurium*) and use thereof.

### BACKGROUND

Microorganisms play an increasingly important and irreplaceable role in solving human problems related food, energy, health, resources, and environmental protection, and also bring huge social and economic benefits to human beings. In order to acquire high-yield, low-consumption, and high-quality strains, mutation breeding is often conducted through physical, chemical, or biological factors. The physical mutagenesis has received widespread attention due to its advantages such as high safety, convenient operation, simple equipment, and high stability. For example, the biochemical degradation ability of activated sludge can be enhanced by changing a microbial composition of the activated sludge through ultraviolet (UV) radiation and optimizing a growth environment of target bacteria, and a mutant with more beneficial mutations that can be stably inherited by offspring can be obtained by ion implantation mutagenesis. A mutagenesis mechanism can be comprehensively and systematically studied through the femtosecond laser technology in combination with physical and biochemical reaction processes. Among them, the ultrasonic mutagenesis has received more and more attention due to its prominent directionality, strong penetrability, high safety, and easy control.

The research on ultrasonic mutagenesis mainly focuses on its biological effects, and there are few studies on its mutagenesis mechanism, which is not conducive to the further development of ultrasonic mutagenesis technology and ultrasonic equipment. The research on the mutagenesis mechanism relies on the accurate analysis of gene variation. The high-energy effects of physical fields such as ultrasonic fields can cause variations in DNA strands, and the variations are mainly divided into the following three types: 1. single nucleotide polymorphism (SNP); 2. InDel, which refers to an insertion or deletion of a small fragment sequence with a length usually of 50 bp or less at a specified position in genome; and 3. large structural variation (SV), which is common, including an insertion or deletion of a long fragment sequence with a length of 50 bp or more, a chromosome inversion, a sequence translocation within a chromosome or between chromosomes, a copy number variation, and some more complex variations. Type 2 and Type 3 variations are commonly referred to as genomic SV In recent years, researchers have found that SVs have a greater impact on a genome than SNPs, SVs on a genome seem to be more suitable than SNPs for explaining the characteristics of species diversity, and some rare and identical SVs are often correlated with the occurrence of some diseases or even cause these diseases.

*S. typhimurium* is a non-adaptive or pantropic *Salmonella* species with a wide host spectrum, which is one of the main pathogenic bacteria causing acute gastroenteritis and is also a very important bacterial species in the development of microbial genetics. *S. typhimurium* is often modified into a live attenuated vaccine vector that is widely used in the research on immunization of various viruses, bacteria, parasites, and the like, which has achieved prominent results. The histidine-auxotrophic *S. typhimurium* reverse mutation assay (Ames test) can be used to detect the potential carcinogenicity of a chemical substance, which is a prominent method to observe a gene mutation and is widely used to screen the genetic toxicity and potential carcinogenicity of a chemical substance or a physical field. It is reported that the Ames test exhibits detection rates of 69.0% and 87.5% for carcinogens in rats and rhesus monkeys, respectively, and is also an important basis for a genetic toxicity screening database established by the quantitative structure-activity relationship (QSAR) of compounds.

Genes involved in histidine synthesis mainly include 9 genes such as hisA, hisB, hisC, *hisD,* hisE, hisF, hisG, hisH, and hisI. The mutation of these genes may cause the loss of histidine synthesis ability, and the normal gene functions of some mutated genes can be restored through reverse mutation, which is the molecular biology basis for the Ames test. Depending on the different types of gene reverse mutations, a variety of strains have been developed for the detection of mutation types. For point mutations or oligonucleotide strand (≤ 4 bp) mutations, four histidine-auxotrophic *S. typhimurium* strains (TA1535, TA1537/TA97/TA97a, TA98, and TA100) for detecting a base substitution or frameshift mutation at a guanine-cytosine (G-C) site in a histidine target gene and a histidine-auxotrophic *S. typhimurium* strain (TA102) for detecting a base substitution or frameshift mutation at an adenine-thymine (A-T) site in a histidine or tryptophan gene are developed, and for error-prone repair mutations caused by large-scale DNA damage, an SOS repair-based Umu test strain (TA1535) is developed. At present, there is no method for detecting an insertion or deletion of an oligonucleotide strand with a length of more than 4 bp in the *S. typhimurium* reverse mutation detection system, which is of great significance for the optimization and evaluation of physical field mutagenesis such as ultrasonic mutagenesis.

### SUMMARY

In view of the deficiencies in the prior art, the present disclosure provides an InDel molecular marker of a *hisD* gene of ultrasonically-mutagenized *S. typhimurium* and use thereof. In the present disclosure, through ultrasonic mutagenesis to *S. typhimurium* and sequencing of the *hisD* gene, it is found that all InDel mutations occur in a core mutation region of the *hisD* gene. Therefore, the core mutation region is used as the InDel molecular marker to determine an insertion or a deletion of a gene reverse mutation, and the InDel molecular marker can also be used to analyze a relationship between a sequence of the *hisD* gene and a function of a protein encoded thereby.

The present disclosure provides an InDel molecular marker of a *hisD* gene of ultrasonically-mutagenized *S. typhimurium,* where the InDel molecular marker is used to determine a type of an oligonucleotide insertion or deletion in an InDel core mutation region of the *hisD* gene, and the InDel molecular marker is a sequence from an 832^{nd} base to a 915^{th} base in the *hisD* gene of the *S. typhimurium* strain, and has a nucleotide sequence set forth in SEQ ID No: 4.

Further, the InDel molecular marker is amplified by the following specific molecular marker primers having the following sequences:
*hisDRC* (F): 5'-GTCAGGTCAGCCAGCGTCT-3' (SEQ ID No: 20); and
*hisDRC* (R): 5'-GTAATCGCATCCACCAAATC-3' (SEQ ID No: 21).

The present disclosure also provides primers for detecting the InDel molecular marker described above, and nucleotide sequences of the primers are as follows:
*hisDRC* (F): 5'-GTCAGGTCAGCCAGCGTCT-3' (SEQ ID No: 20); and
*hisDRC* (R): 5'-GTAATCGCATCCACCAAATC-3' (SEQ ID No: 21).

The present disclosure also provides use of the InDel molecular marker of the *hisD* gene of the ultrasonically-mutagenized *S. typhimurium,* which specifically includes using the molecular marker primers provided by the present disclosure to determine an insertion or a deletion of a gene reverse mutation.

The present disclosure also provides use of the InDel molecular marker of the *hisD* gene of the ultrasonically-mutagenized *S. typhimurium* described above in the direct analysis of a relationship between a sequence of the *hisD* gene and a function of a protein encoded by the sequence of the *hisD* gene.

The present disclosure also provides a method for detecting an oligonucleotide insertion or deletion in a *hisD* gene, including using the specific primers described above to amplify the InDel molecular marker to obtain an amplification product, where the nucleotide sequences of the primers are as follows:
*hisDRC* (F): 5'-GTCAGGTCAGCCAGCGTCT-3' (SEQ ID No: 20), and
*hisDRC* (R): 5'-GTAATCGCATCCACCAAATC-3' (SEQ ID No: 21).

Further, in the method, with a DNA amplification product of a *hisD* gene of histidine-auxotrophic *S. typhimurium* as a control, polyacrylamide gel electrophoresis (PAGE) is performed, and if there is a difference in size between bands of the amplification product and the control, a reverse mutation of the oligonucleotide insertion or deletion occurs.

Further, in the method, the amplification product is sequenced to analyze a specific oligonucleotide sequence of the oligonucleotide insertion or deletion.

The present disclosure also provides use of the method described above in the detection of a reverse mutation in *S. typhimurium* mutagenized by a physical field selected from the group consisting of a pulsed electric field (PEF) and a femtosecond laser.

The present disclosure also provides a kit for detecting the InDel molecular marker described above, where the kit includes the primers for detecting the InDel molecular marker.

### Compared with the prior art, the present disclosure has the following beneficial effects.

In the present disclosure, based on the *hisD* gene sequencing data of 1,752 *S. typhimurium* revertants, a core mutation region of oligonucleotide insertions or deletions of the *hisD* gene is mapped, and then a molecular marker of the core mutation region is designed by narrowing the amplification range, and the PAGE method can be used to effectively determine a type of an insertion or deletion of an oligonucleotide strand of more than 4 bp in the *hisD* gene and accurately analyze a gene variation caused by an ultrasonic physical field, which is conducive to the further exploration of the mutagenesis mechanism.

Compared with the traditional sequencing method, the method for detecting an oligonucleotide insertion or deletion in the InDel core mutation region of the *hisD* gene provided by the present disclosure leads to a more intuitive result, and has a lower test cost and a higher application value.

In addition, the method for detecting an oligonucleotide insertion or deletion in the InDel core mutation region of the *hisD* gene provided by the present disclosure also has guiding significance for the detection of a reverse mutation of *S. typhimurium* mutagenized by a physical field such as PEF and femtosecond laser.

### BRIEF DESCRIPTION OF THE DRAWINGS

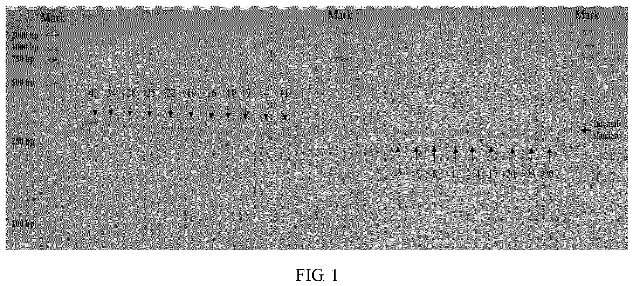

FIG. 1 is an electrophoretogram of all frameshift mutations (oligonucleotide insertions or deletions) in a core mutation region of *hisD* in TA98 revertants, where a PCR amplification product of *hisD* gene of a TA98 strain is used as an internal standard, and meanings of unique oligonucleotide insertion or deletion bands caused by ultrasound are shown in Table 1.

### DETAILED DESCRIPTION OF THE EMBODIMENTS

The present disclosure will be further described below in conjunction with the accompanying drawings and specific examples, but the protection scope of the present disclosure is not limited thereto.

Unless defined otherwise, all terms used in the present disclosure have the same meanings as commonly understood by one of ordinary skill in the art. In the following examples, various processes and methods that are not described in detail are conventional methods known in the art. The sources, trade names, and components needing to be listed of reagents are indicated when the reagents appear for the first time, and unless otherwise specified, the same reagents appearing thereafter are the same as those indicated for the first time. Unless otherwise specified, these reagents are all commercially available.

The equipment used for the ultrasonic treatment of the present disclosure is not limited. The TA98 strain of the present disclosure is purchased from MOLTOXTM Molecular Toxicology Inc., USA. The wild type (WT) *S. typhimurium* is a type of TA98 revertant, which has one more C base than the TA98 strain. The TA98 revertant is *S. typhimurium* that restores the histidine synthesis function.

### Example 1: Detection of the hisD gene and determination of the InDel molecular marker

### (1) Preparation of a bottom medium

16 mL of a phosphate stock solution, 20 mL of a 40% (w/v) glucose solution, and 0.6 mL of a 2 mM histidine-biotin solution were added in turn to 400 mL of a sterilized 1.5% (mass fraction) agar medium (80°C to 90°C), the resulting mixture was thoroughly mixed and used to prepare culture plates (75 mm plate) with 10 mL per plate, and after the mixture was condensed and solidified, the plates were invertedly incubated at 37°C for 24 h.

(2) A TA98 bacterial suspension without ultrasonic treatment was adopted as a control group (TA98 was purchased from MOLTOXTM Molecular Toxicology Inc., USA), and a TA98 bacterial suspension undergoing ultrasonic treatment was adopted as an ultrasonic treatment group. A specific treatment method for the ultrasonic treatment group was as follows: 0.1 mL of a TA98 bacterial suspension undergoing ultrasonic treatment (a seed bacterial suspension was cultivated for 6 h and then subjected to ultrasonic treatment for 5 min at an ultrasonic power density of 20 W/mL and a frequency of 40 kHz) was taken and coated on a plate containing the bottom medium, and then cultivated in a constant-temperature incubator at 37°C for 36 h to 72 h.

876 single colonies of the TA98 revertant resulting from ultrasonic mutagenesis in the ultrasonic treatment group and 876 single colonies of the TA98 revertant resulting from spontaneous mutation in the control group were separately picked (which was conducted through three parallel experiments, with a total of 1,752 colonies) and cultivated in the bottom liquid medium for 12 h at 37°C and 100 r/min, and then the resulting bacterial suspension was collected for PCR amplification (pre-denaturation at 94°C for 3 min, denaturation at 94°C for 20 s, annealing at 55°C for 30 s, and extension at 72°C for 1.5 min, with 35 cycles in total; and extension at 72°C for 10 min). A primer pair for the PCR amplification was as follows:
*hisD* (F): 5'-ATGGAGTAAAGACCATGAGCTTCA-3' (SEQ ID No: 1), and
*hisD* (R): 5'-TGCTTGCTCCTTGAGGGCGT-3' (SEQ ID No: 2).

(3) The PCR product was sent to Sangon Biotech (Shanghai) Co., Ltd. for sequencing, and a multiple sequence alignment tool MAFFT (https://www.ebi.ac.uk/Tools/msa/mafft/) was used to align sequencing results of the 1,752 TA98 revertants with the sequence of the TA98 strain.

The results showed that the frameshift mutations of the TA98 revertants exhibited polymorphic, and there were 127 insertion or deletion mutation types in the ultrasonic treatment group and the control group. These 127 insertion or deletion mutation types were concentrated in a core mutation region (a sequence from an 832^{nd} base to a 915^{th} base of the *hisD* gene, SEQ ID No: 4) of *hisD* gene (full length: 1,305 bp, SEQ ID No: 3). Therefore, the core mutation region was used as an InDel molecular marker, in which a unique oligonucleotide insertion or deletion mutation type appeared after the ultrasonic treatment, as shown in Table 1. The wild type *S*. *typhimurium* in Table 1 was a type of TA98 revertant, which had one more C base than the TA98 strain. Compared with the TA98 strain, base insertion sequences (> 4 bp) of the TA98 revertants caused by ultrasound were all direct repeat sequences.

**Table 1: DNA sequences of core mutation regions (No. 832 to No. 915) in hisD gene of the TA98 revertants**

| (unique oligonucleotide insertion or deletion types of the ultrasonic treatment group) | |
|---|---|
| Type | DNA sequence |
| TA98 | GCCCGCAAGGTGGCGGAGGCGGTAGAACGTCAACTGGCGGAACTGCCGCGCGCGGACACCGC CGGCAGGCCCTGAGCGCCAGTC (SEQ ID No. 5) |
| Wild type | GCCCGCAAGGTGGCGGAGGCGGTAGAACGTCAACTGGCGGAACTGCCGCGCGCGGACACCGC CCGGCAGGCCCTGAGCGCCAGTC (SEQ ID No. 6) |
| +43 bp | GCCCGCAAGGTGGCGGAGGCGGTAGA(ACGTCAACTGGCGGAACTGCCGCGCGCGGACACCG CCGGCAGG)2CCCTGAGCGCCAGTC (SEQ ID No. 7) |
| +34 bp | (GCCCGCAAGGTGGCGGAGGCGGTAGAACGTCAAC)2TGGCGGAACTGCCGCGCGCGGACACC |
| | GCCGGCAGGCCCTGAGCGCCAGTC (SEQ ID No. 8) |
| +28 bp | GCCCGCAAGGTGGCGGAGGCGGTAGAACGTCAA(CTGGCGGAACTGCCGCGCGCGGACACCG) 2CCGGCAGGCCCTGAGCGCCAGTC (SEQ ID No. 9) |
| +25 bp | GCCCGCAAGGTGGCGGAGGCGGTAGAACGTCAAC(TGGCGGAACTGCCGCGCGCGGACAC)₂C GCCGGCAGGCCCTGAGCGCCAGTC (SEQ ID No. 10) |
| +22 bp | GCCCGCAAGGTGGCGGAGGCGGTAGAACGTCAACTGGCGGA(ACTGCCGCGCGCGGACACCG CC)2GGCAGGCCCTGAGCGCCAGTC (SEQ ID No. 11) |
| +19 bp | GCCCGCAAGGTGGCGGAGGCGGTAGAACGTC(AACTGGCGGAACTGCCGCG)2CGCGGACACC GCCGGCAGGCCCTGAGCGCCAGTC (SEQ ID No. 12) |
| +16 bp | GCCCGCAAGGTGGCGGAGGCGGTAGAACGTCAACTGGCGGAACTGCCG(CGCGCGGACACCG CCG)2GCAGGCCCTGAGCGCCAGTC (SEQ ID No. 13) |
| +10 bp | GCCCGCAAGGTGGCGGAGGCGGTAGAACGTCA(ACTGGCGGAA)2CTGCCGCGCGCGGACACC GCCGGCAGGCCCTGAGCGCCAGTC (SEQ ID No. 14) |
| +10 bp | GCCCGCAAGGTGGCGGAGGCGGTAGAACGTCAA(CTGGCGGAAC)2TGCCGCGCGCGGACACC GCCGGCAGGCCCTGAGCGCCAGTC (SEQ ID No. 15) |
| +10 bp | GCCCGCAAGGTGGCGGAGGCGGTAGAACGTCAACTGGCGGAACTGCCGCGCGCGGACACCGC C(GGCAGGCCCT)2GAGCGCCAGTC (SEQ ID No. 16) |
| +7 bp | GCCCGCAAGGTGGCGGAGGCGGTAGAA(CGTCAAC)2TGGCGGAACTGCCGCGCGCGGACACC GCCGGCAGGCCCTGAGCGCCAGTC (SEQ ID No. 17) |
| +7 bp | GCCCGCAAGGTGGCGGAGGCGGTAGAACGTCAACTG(GCGGAAC)2TGCCGCGCGCGGACACC GCCGGCAGGCCCTGAGCGCCAGTC (SEQ ID No. 18) |
| -29 bp | GCCCGCAAGGTGGCGGAGGCGGTAGAACGTCAAC(TGGCGGAACTGCCGCGCGCGGACACCG CC)0GGCAGGCCCTGAGCGCCAGTC (SEQ ID No. 19) |

| | |
|---|---|
| Notes: An underlined sequence represents inserted or deleted bases, + represents insertion, and - represents deletion; and a number at a lower right corner of brackets represents a number of repetitions of a sequence in the brackets, for example, 2 indicates two repetitions and 0 indicates deletion. | |

### Example 2: Use of the InDel molecular marker in the detection of the hisD gene revertant

(1) In order to intuitively and effectively determine a type of an oligonucleotide insertion or deletion (especially a large-fragment DNA insertion or deletion) of *hisD* gene in a TA98 revertant, a characteristic primer pair of the InDel molecular marker was designed according to the core mutation region of the *hisD* gene:
   *hisDRC* (F): 5'-GTCAGGTCAGCCAGCGTCT-3' (SEQ ID No: 20), and
   *hisDRC* (R): 5'-GTAATCGCATCCACCAAATC-3' (SEQ ID No: 21).
(2) The *hisD* gene of the TA98 strain and the *hisD* gene of each of the TA98 revertants obtained in Example 1 were separately subjected to PCR amplification with the primers in step (1), with the PCR procedure being as follows: pre-denaturation at 94°C for 3 min, denaturation at 94°C for 20 s, annealing at 55°C for 30 s, and extension at 72°C for 1.5 min, with 35 cycles in total; and extension at 72°C for 10 min.
(3) The PCR product obtained in step (2) was verified by PAGE.

Preparation of a 6% non-denaturing polyacrylamide (PAM) gel: A cleaned and dried vertical electrophoresis tank was arranged, a 1% agar powder gel was melted in a microwave oven and then injected into a gel tank from two sides of a sealing strip, and the edges were sealed. The reagents were thoroughly mixed according to Table 2 and then poured into the gel tank, a 52-gel hole comb was inserted, the excessive gel was removed, and the gel tank was allowed to stand for 20 min, the unsolidified gel was poured out, an appropriate amount of a 1×TBE (Tris-Borate-EDTA) running buffer was added, and the comb was pulled out, and a syringe needle was used to straighten gel holes, and a 200 mL pipette was used to repeatedly flush the gel holes.

**Table 2: PAGE conditions**

| Electrophoresis conditions | Volume |
|---|---|
| 30% PAM | 8 mL |
| 5×TBE | 8 mL |
| ddH₂O | 23.6 mL |
| 10% Ammonium persulfate (APS) | 400 µL |
| Tetramethylethylene diamine (TEMED) | 40 µL |

The PCR amplification product of *hisD* gene obtained from the TA98 strain (purchased from MOLTOXTM Molecular Toxicology Inc., USA), the PCR amplification product of *hisD* gene obtained from the TA98 revertant prepared in Example 1 as a template, and a 6× loading buffer were thoroughly mixed in a ratio of 3:7:2, 1.0 µL of the resulting mixed sample was added to a spotting well with a pipette, and electrophoresis was conducted at a constant voltage of 220 V for 1.5 h.

After the electrophoresis was completed, the gel was taken out and immersed in a 0.1% (w/v) AgNO₃ aqueous solution (50 mL/gel), and shaken at a low speed for 10 min, the solution was poured off, and the gel was washed twice with an equal amount of distilled water, a developing solution (which was prepared by dissolving 8% (w/v) of glucose, 0.7% (w/v) of boric acid, and 1.1% (w/v) of sodium hydroxide in distilled water, and was used at 50 mL/gel) was added, and the resulting mixture was shaken at a low speed until a clear band (for about 10 min to 20 min) appeared, the developing solution was poured off, and the gel was rinsed with tap water 3 times or more, and a gel imaging system was used for photography, and analysis results were shown in FIG. 1.

FIG. 1 is an electrophoretogram of all oligonucleotide insertion or deletion types in the core mutant region of *hisD* in the TA98 revertants. The PCR amplification product of *hisD* gene of the TA98 strain was adopted as an internal standard, and the meanings of unique oligonucleotide insertion or deletion bands caused by ultrasound were shown in Table 1. It can be seen from the figure that the insertions (+ 1 to + 43 bp) and deletions (- 2 to - 29 bp) of oligonucleotides were +/- distributed above and below a molecular weight (about 372 bp) of the internal standard (the PCR amplification product of *hisD* gene of the TA98 strain). Except for the + 1 bp insertion and - 2 bp deletion, the PAGE electrophoretogram based on the core mutation region can effectively distinguish the band changes caused by insertions or deletions of more than 4 bp, that is, the electrophoretic bands will be distributed above or below the molecular weight (372 bp) of the internal standard.

Therefore, this method can effectively determine a type of an oligonucleotide insertion or deletion of a *hisD* gene of an *S. typhimurium* TA98 revertant (an oligonucleotide insertion type was shown above the molecular weight of the internal standard in the electropherogram and an oligonucleotide deletion type was shown below the molecular weight of the internal standard in the electropherogram) to evaluate an effect of ultrasonic mutagenesis, and can also be used to analyze a relationship between a sequence of the *hisD* gene and a function of a protein encoded thereby.

The above examples are preferred implementations of the present disclosure, but the present disclosure is not limited to the above implementations. Any obvious improvement, substitution, or modification made by those skilled in the art without departing from the essence of the present disclosure should fall within the protection scope of the present disclosure.

## Claims

1. An insertion and deletion (InDel) molecular marker of a *hisD* gene of an ultrasonically-mutagenized *Salmonella typhimurium,* **characterized in that** the InDel molecular marker is a sequence from an 832^{nd} base to a 915^{th} base in the *hisD* gene of the ultrasonically-mutagenized *Salmonella typhimurium,* and has a nucleotide sequence set forth in SEQ ID No: 4.

2. The InDel molecular marker of the *hisD* gene of the ultrasonically-mutagenized *Salmonella typhimurium* according to claim 1, **characterized in that** the InDel molecular marker is amplified by the following specific molecular marker primers having the following sequences:
*hisDRC* (F): 5'-GTCAGGTCAGCCAGCGTCT-3', and
*hisDRC* (R): 5'-GTAATCGCATCCACCAAATC-3'.

3. Primers for detecting the InDel molecular marker of the *hisD* gene of the ultrasonically-mutagenized *Salmonella typhimurium* according to claim 1, **characterized in that** nucleotide sequences of the primers are as follows:
*hisDRC* (F): 5'-GTCAGGTCAGCCAGCGTCT-3', and
*hisDRC* (R): 5'-GTAATCGCATCCACCAAATC-3'.

4. A use of the InDel molecular marker of the *hisD* gene of the ultrasonically-mutagenized *Salmonella typhimurium* according to claim 1 in a determination of an insertion or a deletion of a gene reverse mutation.

5. A use of the InDel molecular marker of the *hisD* gene of the ultrasonically-mutagenized *Salmonella typhimurium* according to claim 1 in an analysis of a relationship between a sequence of the *hisD* gene and a function of a protein encoded by the sequence of the *hisD* gene.

6. A method for detecting an oligonucleotide insertion or deletion in the *hisD* gene, **characterized by** comprising using the primers according to claim 3 to amplify the InDel molecular marker to obtain an amplification product, wherein the nucleotide sequences of the primers are as follows:
*hisDRC* (F): 5'-GTCAGGTCAGCCAGCGTCT-3', and
*hisDRC* (R): 5'-GTAATCGCATCCACCAAATC-3'.

7. The method for detecting the oligonucleotide insertion or deletion in the *hisD* gene according to claim 6, **characterized in that** with a DNA amplification product of a *hisD* gene of a histidine-auxotrophic *Salmonella typhimurium* as a control, a polyacrylamide gel electrophoresis is performed, and if there is a difference in size between bands of the amplification product and the control, a reverse mutation of the oligonucleotide insertion or deletion occurs.

8. The method for detecting the oligonucleotide insertion or deletion in the *hisD* gene according to claim 7, **characterized in that** the amplification product is sequenced to analyze a specific oligonucleotide sequence of the oligonucleotide insertion or deletion.

9. A use of the method according to any one of claims 6 to 8 in a detection of a reverse mutation in *Salmonella typhimurium* mutagenized by a physical field selected from the group consisting of a pulsed electric field and a femtosecond laser.

10. A kit for detecting the InDel molecular marker of the *hisD* gene of the ultrasonically-mutagenized *Salmonella typhimurium* according to claim 1, **characterized in that** the kit comprises the primers for detecting the InDel molecular marker according to claim 3.
